# EUROPÄISCHE PATENTSCHRIFT

(11) **EP 2 801 345 B1**
(45) Veröffentlichungstag und Bekanntmachung des Hinweises auf die Patenterteilung: **27.02.2019**
(21) Anmeldenummer: 14166936.6
(22) Anmeldetag: 02.05.2014
(51) Int. Cl.: A61K 6/06, A61K 6/00

(54) **Langlebiges Dentalmaterial mit verbesserten transparenten Eigenschaften**
Durable dental material with improved transparent properties
Matériau dentaire de longue durée ayant des propriétés transparentes améliorées

(30) Priorität: 08.05.2013 DE 102013007894
(43) Veröffentlichungstag der Anmeldung: 12.11.2014
(73) Patentinhaber: Kulzer GmbH, 63450 Hanau (DE)
(72) Erfinder: Ruppert, Klaus, 63477 Maintal (DE); Hohmann, Alfred, 61389 Schmitten (DE)
(74) Vertreter: Bendele, Tanja

(56) Entgegenhaltungen:
- EP-A2- 0 518 454
- US-A1- 2002 156 152
- US-A1- 2005 252 415
- US-B1- 6 730 156

## Beschreibung

Es wird ein Dentalmaterial offenbart, welches umfasst (a) mindestens eine härtbare Monomer- und/oder Polymerkomponente, und (b) mindestens eine Füllstoffkomponente umfassend agglomerierte Oxidpartikel mit einer Matrix und einer Dotierkomponente, wobei die Matrix Siliziumdioxid enthält und die Dotierkomponente Zirkoniumdioxid umfasst, wobei die agglomerierten Oxidpartikel in der gehärteten Polymermatrix des Dentalmaterials eingebunden sind und bei einem Abrasionsprozess nur schichtweise zusammen mit der Polymermatrix des Dentalmaterials abgetragen werden und nicht als einzelne, vollständige Partikel herausbrechen. Ferner weist das gehärtete Dentalmaterial eine hohe Transparenz auf.

Zur Erfüllung hoher ästhetischer Ansprüche müssen Dentalkomposite wie Füllungskomposite oder Verblendkomposite eine hohe Transparenz aufweisen. Diese Transparenz wird in der Regel durch eine optimale Anpassung der Brechzahlen der Füllstoffe und der Polymermatrix erreicht. Hierbei sind jedoch aufgrund verschiedener physikalischer und chemischer Randbedingungen sowohl bei der Auswahl der Füllstoffe als auch der Monomere sehr enge Grenzen gesetzt.

Glasfüllstoffe haben typischerweise Brechzahlen zwischen 1,50 und 1,54. Viele geeignete Monomere für Dentalmaterialien haben ebenfalls eine Brechzahl, die in diesem Bereich liegt. Komposite, welche ausschliesslich Glasfüller enthalten, weisen aber ungünstige Handlingseigenschaften auf. Aus diesem Grund werden diesen Zubereitungen in der Regel Rheologiemodifizierer wie z.B. pyrogene Kieselsäuren ("Aerosile") zugesetzt. Die pyrogenen Kieselsäuren haben jedoch eine Brechzahl von 1,46 und liegen damit weit von der optimalen Brechzahl (> 1,50) für Dentalmaterialien entfernt. Mit der Zugabe dieser Rheologiemodifizierer verschlechtert sich daher die Transparenz des daraus hergestellten Dentalmaterials.

EP 1 227 781 B1 offenbart ein Dentalmaterial eines härtbaren Harzes mit Siliziumdioxidteilchen, die in dem Harz verteilt sind sowie mindestens einem Schwermetalloxid, wobei die Siliziumoxidteilchen einen durchschnittlichen Durchmesser von weniger als 200 nm haben und in einer Menge vorhanden sind, die grösser als 40 Gew.-% ist, basierend auf dem Gewicht des Dentalmaterials. Das Schwermetalloxid wird als separates Sol zugesetzt. EP 1225867B1 offenbart ein Dentalmaterial mit Nicht-Schwermetalloxidteilchen mit einem durchschnittlichen Durchmesser von kleiner 300 nm und einem säuremodifizierten Schwermetalloxid in einem härtbaren Harz. EP1229886B1 offenbart ein weiteres Füllmaterial für Dentalmaterialien. Das Füllmaterial ist ein amorphes Cluster mit einem Kristallinitätsindex von kleiner 0,1 umfassend ein Nicht-Schwermetalloxid und ein amorphes Schwermetalloxid in Teilchenform mit einem Durchmesser kleiner gleich 100 nm. EP0518454A2 thematisiert, dass ein Dentalmaterial mit Zirkondioxid mit einer Teilchengrösse oberhalb 1 µm eine ungenügende Deckfähigkeit aufweist.

US6730156B1 offenbart einen Füllstoff, welcher einen im Wesentlichen amorphen Cluster aus Siliziumdioxid-Partikeln mit einem Durchmesser von weniger als 40 nm und Zirkoniumdioxid-Partikel mit einem Durchmesser von weniger als 10 nm umfasst und in ein härtbares Harz gemischt werden kann.

US2005252415A1 offenbart dentale Füllstoffe umfassend mit Oxyfluoriden beschichtete Siliziumdioxid- und Zirkoniumdioxid-Partikel.

US2002156152A1 offenbart ein Dentalmaterial umfassend ein härtbares Harz und in diesem dispergierte Siliziumdioxid-Partikel mit einem Durchmesser von weniger als 200 nm.

EP0518454A2 offenbart die Verwendung eines opaken Dentalwerkstoffs, welcher ein Matrix bildendes Glaspulver enthaltend Siliziumdioxid und als Trübungsmittel Zirkoniumdioxid umfasst.

Der Erfindung liegt die Aufgabe zu Grunde einen Rheologiemodifizierer anzugeben, welcher nicht zu einer Verschlechterung der Transparenz des daraus hergestellten Komposits führt, wie dies für Siliciumdioxid bekannt ist. Ferner wird eine ausreichende Deckfähigkeit benötigt, so dass ein Material bereitgestellt werden soll, dass eine Balance zwischen einer gewünschten Transparenz und gewissen Deckfähigkeit als Rheologiemodifizierer bietet. Eine weitere Aufgabe besteht darin einen Rheologiemodifizierer anzugeben, welcher zusätzlich noch die Abrasionseigenschaften des daraus hergestellten Dentalkomposits günstig beeinflusst. Als günstig wird es angesehen, wenn bei einer Abrasion ein möglichst geringes Tiefenprofil erzeugt wird, d.h. ein möglichst geringes Volumen abgetragen wird, die Rauigkeit des Dentalmaterials nach vollständiger Aushärtung gering ist und durch Abrasion möglichst kaum zunimmt und/oder das Reflexionsvermögen möglichst unverändert bleibt. Eine geringe Rauigkeit minimiert eine Plaqueaffinität. Das Reflexionsvermögen soll sich selbst nach starker Beanspruchung über einen langen Zeitraum möglichst nicht oder nur gering verändern. Auch die Glanzstabilität, d.h. die Differenz der Glanzzahl vor und nach eines Abrasionsexperiments, des gehärteten Dentalmaterials soll deutlich verbessert werden.

Erfindungsgemäss wird die Aufgabe durch Verwendung einer spezifischen ZrO₂ dotierten Kieselsäure als Reologiemodifizierer in der Füllstoffkomponente gelöst. Der ZrO₂-Gehalt in dem Oxidgemisch des Reologiemodifizieres in der Füllstoffkomponente wird dabei zur Anpassung der Brechzahl im Bereich von ca. 5 - 25 Gew. % ZrO₂ eingestellt.

Die Aufgabe der Erfindung wurde gemäss Anspruch 1 der Erfindung sowie der Ansprüche 10-11 und 14 gelöst. Bevorzugte Ausführungsformen sind in den Unteransprüchen und in der Beschreibung detailliert beschrieben.

Die vorgenannten Aufgaben werden erfindungsgemäss durch die Verwendung mindestens einer Füllstoffkomponente umfassend agglomerierte Primärpartikel mit einer durchschnittlichen Korngrösse der Agglomerate von ca. 1 - 10 µm gelöst. Diese Füllstoffe werden bei Abrasion nicht als Gesamtpartikel aus der Polymermatrix herausgerissen, sondern schichtweise abgetragen. Hierdurch kommt es nicht zu einer Erhöhung der Oberflächenrauigkeit, einem verminderten Glanz und einer erhöhten Plaqueaffinität.

Erfindungsgemäss werden die Aufgaben z.B. durch die Verwendung der Produkte ZirkonSil 520 und ZirkonSil 535 gelöst. Besonders vorteilhaft ist es, wenn die agglomerierten Oxidpartikel umfassend Siliziumdioxid und Zirkoniumdioxid mit einem organofunktionellen einpolymerisierbaren Silan oberflächenfunktionalisiert werden. Dabei ist ein gegenüber den organischen Monomeren und Polymeren reaktives organofunktionelles Silan, vorzugsweise ein olefinisches Alkoxysilan, wie Methacryloxy-funktionalisiertes oder Vinyl-funktionelles Alkoxysilan besonders bevorzugt. Die Silanisierung kann in-situ während der Herstellung des Dentalmaterials erfolgen oder auch zuvor in einem separaten Schritt. Im zweiten Fall werden die agglomerierten Oxidpartikel (Agglomerate) der Erfindung in einem separaten Schritt mit einem organofunktionellen Silan oberflächenmodifiziert. Die so modifizierten agglomerierten Oxidpartikel können sodann als Reologiemodifizierer in der Füllstoffkomponente dem Dentalmaterial zugesetzt werden.

Gegenstand der Erfindung ist ein Dentalmaterial, umfassend (a) mindestens eine härtbare Monomer- und/oder Polymerkomponente, und (b) mindestens eine Füllstoffkomponente umfassend agglomerierte Oxidpartikel mit einer Matrix und einer Dotierkomponente, wobei die Matrix Siliziumdioxid enthält und die Dotierkomponente Zirkoniumdioxid umfasst. Die agglomerierten Oxidpartikel (Agglomerate) werden als Rheologiemodifizierer in der Füllstoffkomponente zugesetzt.

Erfindungsgemäss ist es weiter bevorzugt, wenn die Dotierkomponente Zirkoniumdioxid in mindestens einer Domäne, also in mindestens einem Bereich der Oxidpartikel vorliegt. Weiter kann es bevorzugt sein, wenn die Dotierkomponente zumindest teilweise kristallin in der Siliziumdioxidmatrix vorliegt. Die Dotierkomponente ist darüber hinaus zumindest teilweise in der Matrix des Siliziumdioxids eingebettet. Vorzugsweise bildet die Dotierkomponente mindestens eine kristalline Domäne in den Primärpartikeln aus.

Die agglomerierten Oxidpartikel umfassen im Wesentlichen agglomerierte Primärpartikel, insbesondere mit einer Matrix aus Siliziumdioxid. Die Oxidpartikel, insbesondere die Primärpartikel sind mit Zirkoniumdioxid dotiert, bevorzugt mit mikrokristallinem Zirkoniumdioxid. Vorzugsweise liegt die Dotierkomponente als mindestens eine Domäne vor, insbesondere als mindestens eine kristalline Domäne. Bevorzugt ist ferner ein Dentalmaterial in dem die agglomerierten Oxidpartikel Agglomerate von Siliziumdioxid Primärteilchen umfassen, die mit Zirkoniumdioxid dotiert sind, wobei die Agglomerate eine Partikelgrösse von grösser gleich 0,1 bis kleiner gleich 12 µm aufweisen, insbesondere von 0,6 bis 12 µm, bevorzugt beträgt der mittlere Partikeldurchmesser der Agglomerate 2,6 µm bis 3,5 µm, um 2,6 µm oder um 3,5 µm gemessen über eine Volumenverteilung (volume-weighted) in einer Aerosol Dispersion. Die Agglomerate enthalten Primärteilchen aus Oxidpartikeln von Siliziumdioxid, die mit Zirkoniumdioxid dotiert sind.

Die erfindungsgemässen Dentalmaterialien sind besonders ästhetisch und wirken besonders natürlich, da der Brechungsindex der agglomerierten Oxidpartikel zwischen 1,49 bis 1,55 liegt. Besonders bevorzugt sind daher agglomerierte Oxidpartikel mit einem Brechungsindex von 1,49 bis 1,55, insbesondere von 1,50 bis 1,53, besonders bevorzugt von 1,51 bis 1,53, vorzugsweise von 1,516 bis 1,524.

Nach einer Ausführungsform der Erfindung sind Dentalmaterial und Füllstoffkomponenten bevorzugt, die agglomerierte Oxidpartikel von Siliziumdioxid enthaltenden Primärteilchen sind, wobei die Primärteilchen Zirkoniumdioxid-Domänen enthalten. Entsprechend einer Ausführungsform der Erfindung sind Dentalmaterial und Füllstoffkomponenten besonders bevorzugt, die agglomerierte Oxidpartikel von Siliziumdioxid enthaltenden Primärteilchen sind, wobei die Primärteilchen mikrokristalline Domänen (synonym zu Domaine) von 4 bis 7 nm umfassen. Vorzugsweise weisen die Oxidpartikel mikrokristalline Zirkoniumdioxid enthaltende Domänen auf, vorzugsweise Domänen bestehend aus Zirkoniumdioxid, besonders bevorzugt mikrokristalline aus Zirkoniumdioxid bestehende Domänen. Zudem ist es bevorzugt, wenn die agglomerierten Oxidpartikel der Primärteilchen eine spezifische Oberfläche von 5 bis 10 m²/g aufweisen.

Entsprechend einer weiteren Ausführungsform ist es bevorzugt, wenn das Dentalmaterial oder die Füllstoffkomponente agglomerierte Oxidpartikel umfassend die Matrix und die Dotierkomponente einem Gemisch von Metalldioxiden ausgewählt aus Siliziumdioxid und Zirkoniumdioxid entspricht.

Es hat sich ferner gezeigt, dass das Dentalmaterial oder die Füllstoffkomponente die erfindungsgemässen Aufgaben, wie die Anforderungen an die Transparenz, den geringen Abrieb sowie die geringe Rauigkeit besonders gut lösen, wenn die agglomerierten Oxidpartikel in Bezug auf ihre Gesamtzusammensetzung 1 bis 25 Gew.-% Zirkoniumdioxid umfassen, d.h. in den Oxidpartikeln kann die Dotierkomponente von 1 bis 25 Gew.-% in Bezug auf die Gesamtzusammensetzung (100 Gew.-%) des Oxidpartikels betragen, bevorzugt beträgt der Gehalt an Zirkoniumdioxid 10 bis 25 Gew.-%, vorzugsweise 10 bis 15 Gew.-%.

Besonders bevorzugte agglomerierte Oxidpartikel umfassen in Bezug auf ihre Gesamtzusammensetzung 85 bis 90 Gew.-% Siliziumdioxid und 10 bis 15 Gew.-% Zirkoniumdioxid, wobei es weiter bevorzugt ist, wenn die Primärteilchen der agglomerierten Oxidpartikel mikrokristalline Domänen von 4 bis 7 nm umfassen und vorteilhaft der Kristallinitätsindex 0.6 bis 0.7 - bestimmt nach der Methode von Windisch et al. (WO 01/30306A) beträgt und die agglomerierten Oxidpartikel mit mindestens einem gegenüber mindestens einer Monomer- und/oder Polymerkomponente reaktiven organofunktionellen Silan oberflächenmodifiziert vorliegt. Diese erfindungsgemäss behandelten agglomerierten Oxidpartikel weisen hervorragende Eigenschaften in Abrasionsmessungen, bezüglich der Glanzzahl, eine hervorragende Transparenz und sehr gute Werte bei Messungen der Reflexion und Rauheit nach einem Zahnbürstentest auf.

Ebenso sind Dentalmaterialen umfassend mindestens eine Füllstoffkomponente oder Füllstoffkomponenten umfassend die agglomerierten Oxidpartikel bevorzugt, in denen die agglomerierten Oxidpartikel in Bezug auf die Gesamtzusammensetzung des Dentalmaterials von 5 bis 30 Gew.-%, bevorzugt zu 5 bis 20 Gew.-%, vorzugsweise 10 bis 25 Gew.-%, 15 bis 25 Gew.-%, 20 bis 80 Gew.-%, besonders bevorzugt 20 bis 30 Gew.-%, vorzugsweise 15 bis 25 Gew.-%, alternativ etwa 20 Gew.-% mit einem Schwankungsbereich von plus/minus 2,5 Gew.-% vorliegen. Gegenstand der Erfindung sind Dentalmaterialen umfassend mindestens eine Füllstoffkomponente oder Füllstoffkomponenten umfassend die agglomerierten Oxidpartikel bevorzugt, in denen die agglomerierten Oxidpartikel in Bezug auf die Gesamtzusammensetzung des Dentalmaterials von 5 bis 30 Gew.-%. Gegenstand der Erfindung sind somit Dentalmaterialien und/oder Füllstoffkomponenten umfassend agglomerierte Oxidpartikel einer ZrO₂-dotierten Kieselsäure. Hervorragende Ergebnisse werden mit einem Gehalt von 10 bis 25 Gew.-% agglomerierte Oxidpartikel im Dentalmaterial erzielt. Der deutliche Vorteil der Dotierung gegenüber einer Beschichtung oder blossen Mischung mit Zirkoniumdioxid spiegelt sich in der hohen Transparenz und dem guten Reflexionsvermögen sowie der geringen Rauigkeit selbst nach einem Zahnbürstentest wieder. Eine Kieselsäure, die mit Zirkoniumdioxid beschichtet ist, wie das Produkt JE340 zeigt deutlich schlechtere Ergebnisse bei der Transparenz, der Reflexion und Rauigkeit im Anschluss an einen Zahnbürstentest.

Weiter sind Dentalmaterial umfassend Füllstoffkomponenten oder Füllstoffkomponenten bevorzugt, die agglomerierte Oxidpartikel mit einem molaren Verhältnis von Siliziumdioxid und Zirkoniumdioxid von 1 zu 9 aufweisen, insbesondere mit einem molaren Verhältnis von 1 zu 8 bis 1 zu 6.

Ebenso ist ein Dentalmaterial mit einer Füllstoffkomponente oder eine Füllstoffkomponente bevorzugt, die agglomerierte Oxidpartikel umfassend Agglomerate von Primärteilchen mit Siliziumdioxid und Zirkoniumdioxid umfasst, wobei die Agglomerate eine Partikelgrösse d₅₀ von 0,5 bis 12 µm, erfindungsgemäss von 0,5 bis 10 µm, insbesondere größer gleich 1 bis etwa 9 µm aufweisen, insbesondere liegt der d₅₀ im Bereich von 1,5 bis 5 µm, bevorzugt ist d₅₀ im Bereich von 2 bis 4 µm, besonders bevorzugt d₅₀ im Bereich von 2,6 bis 3,5 µm. Ferner ist es bevorzugt, wenn die agglomerierten Oxidpartikel der Füllstoffkomponente eine Korngrössenverteilung aufweisen von d₉₀ kleiner gleich 12 µm und eine mittlere Partikelgrösse d₅₀ etwa zu 2,4 bis 3,0 µm.

Weiter ist es bevorzugt, wenn die agglomerierten Oxidpartikel, insbesondere das Agglomerat, weiter bevorzugt die agglomerierten Primärpartikel mit organofunktionellen Silanen oberflächenmodifiziert sind und somit zumindest teilweise vorteilhaft im Wesentlichen alle Primärpartikel in das gehärtete Dentalmaterial einpolymerisiert, insbesondere mehrfach kovalent mit dem umgebenden Polymer gebunden, vorliegen. Je nach Herstellmethode liegen die agglomerierten Oxidpartikel auch als Aggregate vor. Ein Vorteil der erfindungsgemäss verwendeten Oxidpartikel ist, dass diese nicht durch hohe Scherkräfte, z.B. während des Herstellungsprozesses, in die Primärteilchen aufgespalten werden. Insbesondere werden jeweils unabhängig weder (a) die agglomerierten Oxidpartikel, (b) die agglomerierten und aggregierten Oxidpartikel und/oder (c) die oberflächenmodifizierten agglomerierten Oxidpartikel, insbesondere mit Umsetzungsprodukten von organofunktionellen Silanen modifizierte Primärteilchen nicht durch hohe Scherkräfte, die während der Herstellung von Dentalmaterialien auftreten, in die Primärpartikel aufgespalten.

Diese hohen Scherkräfte treten insbesondere im Drei-Walzenstuhl, Zentrifugalmischer, Planetenmischer oder Dissolver, etc. auf, die während der Herstellung von Dentalmaterialien, wie z.B. Kompositen, in der Regel eingesetzt werden. Somit liegen die agglomerierten Oxidpartikel der Erfindung vorteilhaft auch als aggregierte Oxidpartikel vor, dabei kann die Aggregation der Primärpartikel durch oxidische Bindungen direkt bei der Herstellung der Oxidpartikel und/oder durch die Silanisierung eingestellt werden.

Gegenstand der Erfindung ist auch ein Dentalmaterial oder eine Füllstoffkomponente umfassend agglomerierte Oxidpartikel mit einer Oberflächenmodifizierung, insbesondere einer hydrophoben Oberflächenmodifizierung, vorteilhaft durch eine Oberflächenbelegung mit einem organofunktionellen Silan. Bevorzugte Silane sind gegenüber der Monomer- und/oder Polymerkomponente und den Oxidpartikeln reaktive organofunktionelle Silane, wie olefinisch-funktionalisierte Alkoxysilane, wie lineare, verzweigte und/oder cyclische Alkenyl-, (Meth)arcylat-, oder Urethanfunktionalisierte Alkoxsilane oder deren Hydrolyse- und/oder Kondensationsprodukte. Die organofunktionelle Gruppe der Alkoxysilane umfasst vorteilhaft 2 bis 20 C-Atome, insbesondere 2 bis 10 C-Atome die von Heteroatomen unterbrochen oder mit diesen substituiert sein können.

Bevorzugte organofunktionalisierte Alkoxysilane oder deren Umsetzungsprodukte, insbesondere deren Hydrolyse- und/oder Kondensationsprodukte mit denen die agglomerierten Oxidpartikel, vorzugsweise die Primärpartikel, oberflächenmodifiziert sind, umfassen Methacryloxyalkylentrialkoxysilan, wobei die bifunktionelle AlkylenGruppe 1 bis 8 C-Atome aufweist, 3-Methacryloxytrimethoxysilan, 3-Methacryloxytriethoxysilan, 3-Methacryloxypropyltriethoxysilan, 3-Methacryloxypropyltrimethoxysilan, alpha-Methacryloxypropyltrimethoxysilan.

Gegenstand der Erfindung ist ein Dentalmaterial enthaltend: (a) mindestens eine härtbare Monomer- und/oder Polymerkomponente, (b) mindestens eine Füllstoffkomponente umfassend (b.1) mindestens agglomerierte Oxidpartikel, insbesondere als Rheologiemodifizierer, mit einer Matrix und einer Dotierkomponente, wobei die Matrix Siliziumdioxid umfasst und die Dotierkomponente Zirkoniumdioxid, insbesondere beträgt (b.1) von 5 bis 30 Gew.-%, (b.2) Gläser, insbesondere Dentalgläser von 30 bis 65 Gew-%, vorzugsweise 30 bis 60 Gew.-%, und optional (b.3) weitere Rheologiemodifizierer, die häufig auch als Füllstoffe und umgekehrt aufgeführt werden, wie Kieselsäure, Siliziumdioxid, Pyrogene Kieselsäure, insbesondere 0 bis 5 Gew.-%, vorzugsweise 0 bis 1 Gew.-%, und/oder Gemische von mindestens zwei der vorgenannten Füllstoffkomponenten, und optional (c) mindestens einen Initiator, insbesondere bis 1 Gew.-%, vorteilhaft von 0,0001 bis 1 Gew.-% und (d) optional mindestens ein Pigment, insbesondere bis 1 Gew.-%, vorzugsweise 0,0001 bis 0,1 Gew.-% in Bezug auf die Gesamtzusammensetzung.

Vorteilhaft umfasst die Füllstoffkomponente die Komponenten b.1, b.2, b.3 in Bezug auf die Gesamtzusammensetzung des Dentalmaterials bis zu 80 Gew.-%, besonders vorteilhaft zu 50 bis 80 Gew.-%, vorzugsweise von 55 bis 80 Gew.-%, bevorzugt von 60 bis 80 Gew.-%, weiter bevorzugt von 65 bis 80 Gew.-%, besonders bevorzugt zu 70 bis 80 Gew.-%, wobei davon die Dentalgläser 30 bis 60 Gew.-% in Bezug auf die Gesamtzusammensetzung, bevorzugt 40 bis 55 Gew.-% ausmachen. Die Gesamtzusammensetzung des Dentalmaterials beträgt 100 Gew.-%.

Erfindungsgemäss ist es bevorzugt keine üblichen Rheologiemodifizierer, die häufig auch als Füllstoffe bezeichnet werden, wie unmodifizierte Kieselsäure, Siliziumdioxid und/oder pyrogene Kieselsäure einzusetzen. Erfindungsgemäss ist es vorzugsweise ausreichend als Füllstoffkomponente ausschliesslich die erfindungsgemässen agglomerierten Oxidpartikel mit Siliziumdioxid Matrix und Zirkoniumdioxid als Dotierkomponenten im Dentalmaterial als Rheologiemodifizierer einzusetzen. Übliche Reologiemodifizierer umfassend unmodifizierte Kieselsäure, Siliziumdioxid und/oder pyrogene Kieselsäure werden allenfalls in sehr geringen Mengen eingesetzt von 0 bis 2,8 Gew.-%, vorzugsweise von 0,001 bis 1,75 Gew.-% eingesetzt. Entsprechend kann vorzugsweise ein Gemisch von (b.1) und (b.2) oder optional ein Gemisch von (b.1), (b.2) und (b.3) im Dentalmaterial eingesetzt werden, insbesondere mit einem Gehalt von bis zu 80 Gew.-% in Bezug auf die Dentalzusammensetzung, wobei die Komponenten (b.1), (b.2) und/oder (b.3) in allen denkbaren Zusammensetzungen vorliegen können. Vorteilhaft liegen die Komponenten (b.1), (b.2) und (b.3) jeweils unabhängig voneinander silanisiert vor.

Weitere Gegenstände der Erfindung sind ein Dentalmaterial mit einer Füllstoffkomponente umfassend agglomerierte Oxidpartikel und die agglomerierten Oxidpartikel selbst, insbesondere mit einer Matrix und einer Dotierkomponente, wobei die Matrix Siliziumdioxid umfasst und die Dotierkomponente Zirkoniumdioxid umfasst, umfassend Agglomerate von Primärteilchen enthaltend Siliziumdioxid und Zirkoniumdioxid, wobei die Primärteilchen im Durchschnitt mindestens einen Partikeldurchmesser von circa 3 bis 70 nm, insbesondere von 10 bis 50 nm (Nanometer) aufweisen. Erfindungsgemäss liegt die Dotierkomponente in Form mindestens einer Domain in der Siliziumdioxidmatrix vor. Reine Gemische von Siliziumdioxid und Zirkoniumdioxid oder ein Siliziumdioxid mit einer äusseren Beschichtung mit Zirkoniumdioxid weisen nicht die erfindungsgemäss erzielten Eigenschaften auf wie es anhand der Beispiele belegt wurde. Bevorzugt besteht die ZrO₂-dotierte Füllstoffkomponente aus agglomerierten Primärpartikeln mit einem überwiegenden Partikeldurchmesser von ca. 10 - 50 nm (Nanometer).Ferner weisen die erfindungsgemässen agglomerierten Oxidpartikel einen Kristallinitätsindex von 0,6 bis 0,7 auf, der nach Windisch et al WO01/30306, US 7,030,049, EP 1229886B1 bestimmt wird.

Das erfindungsgemässe Dentalmaterial kann ein Füllungskomposit, Verblendungskomposit, Grünling eines künstlichen Zahns, Grünling einer Verblendung, Grünling eines Inlays, Grünling eines Implantats, Grünling eines Trägermaterials für eine lokale Freisetzung von pharmazeutisch wirksamen Substanzen, Grünling eines Trägermaterials für eine lokale Antibiotika-Therapie oder ein Grünling eines Fräsblocks zur Herstellung von Zahnersatz nach der CAD/CAM Technik oder zumindest ein Teil der vorgenannten Dentalmaterialien sein. Unter einem Grünling wird in diesem Zusammenhang das vorgeformte und nicht ausgehärtete oder nicht vollständig ausgehärtete Dentalmaterial verstanden. Der aus dem Dentalmaterial vorgeformte Grünling einer Verblendung kann durch anschliessende Härtung vollständig ausgehärtet werden, um bei Bedarf mechanisch weiter bearbeitet werden zu können.

Entsprechend einer weiteren Ausführungsform der Erfindung ist ebenfalls Gegenstand der Erfindung eine Füllstoffkomponente umfassend agglomerierte Oxidpartikel mit einer Matrix und einer Dotierkomponente, wobei die Matrix Siliziumdioxid enthält und die Dotierkomponente Zirkoniumdioxid umfasst, und wobei die agglomerierten Oxidpartikel mit einem organofunktionellen Silan, insbesondere mit dem Umsetzungsprodukt des Silans, oberflächenmodifiziert sind. Unter organofunktionellen Silanen werden die vorstehenden Silane sowie auch deren Umsetzungsprodukte auf der Oberfläche der Oxidpartikel verstanden. Bevorzugt sind die agglomerierten Oxidpartikel mit Umsetzungsprodukten von olefinischen Alkoxysilanen, insbesondere von 3-Methacryloxypropyltrimethoxysilan und/oder 3-Methacryloxypropyltriethoxysilan oberflächenmodifiziert.

Das Dentalmaterial kann erfindungsgemäss insgesamt an (b) Füllstoffkomponenten einem Gesamtfüllstoffgehalt von 20 bis 98 Gew.-%, insbesondere von 70 bis 95 Gew.% aufweisen umfassend (b.1) 10 bis 30 Gew.-%, vorzugsweise 10 bis 25 Gew.-%, besonderes bevorzugt 15 bis 25 Gew.-%, insbesondere 10 bis 15, 15 bis 20 oder alternativ 20 bis 25 Gew.-% agglomerierte Oxidpartikel, und insbesondere silanisierte Oxidpartikel mit einer Silicumdioxidmatrix und Zirkoniumdioxid als Dotierkomponente mit einem Kristallinitätsindex von 0.6 bis 0.7, und/oder (b.2) mindestens ein Dentalglas von 40 bis 60 Gew.-%, wie 45 bis 50 oder 50 bis 65 Gew.-%, wobei vorzugsweise ein Gemisch von Dentalgläsern aus 50 bis 90 % grob- und 10 bis 50 % feinteiligen Dentalgläsern eingesetzt wird, welche ein Grössenverhältnis, bezogen auf die mittlere Partikelgrösse (d₅₀-Wert), von feinteilig zu grobteilig von 1:4 bis 1:30 aufweisen, und optional, (b.3) 0,5 bis 5 Gew.% nichtagglomerierte Nanofüller mit Partikelgrössen von 1 bis 50 nm.

Als (a) härtbare Monomer- und/oder Polymerkomponenten werden bevorzugt für das Dentalmaterial ausgewählt ein oder mehrere Monomere aus der Monomermischung (i), (ii) und (iii):
(i). mindestens ein Monomer der Gruppe Bisglycidylacrylat, alkoxyliertes Pentaerythrioltetraacrylat, TCD-di-HEMA oder TCD-di-HEA, insbesondere zu 5 bis 20 Gew.-%, insbesondere 9 bis 20 Gew.-%, vorzugsweise zu 10 bis 20 Gew.-%, bevorzugt 10 bis 17 Gew.-% bezogen auf die Gesamt zusammensetzung und
(ii). mindestens 5 bis 20 % multifunktionelle Vernetzer UDMA (Diurethandimethacrylat), insbesondere 10 bis 15 Gew.-% bezogen auf die Gesamtzusammensetzung, und
(iii). optional Rest TEDMA (Trimethylenglykoldimethacrylat) und/oder weitere multifunktionelle Vernetzer, insbesondere 0 bis 5 Gew.-%, vorzugsweise 0,001 bis kleiner gleich 3 Gew.-% in Bezug auf die Gesamtzusammensetzung,
wobei (i), (ii) und (iii) zu insgesamt 5 bis 35 Gew.-% im Dentalmaterial vorliegen, vorzugsweise 15 bis 35 Gew.-%, besonders bevorzugt 20 bis 35 Gew.-%, weiter bevorzugt 20 bis 25 Gew.-%,
c) bis 1 % Initiator(en) und
(b.2) optional in der Füllstoffkomponente mindestens ein weiteres Dentalglas mit sich von den grob- und feinteiligen Dentalgläsern unterscheidender Partikelgrösse, wobei der Anteil an (i) Monomeren in der Gesamtzusammensetzung 9 oder 10 bis 17 Gew.-% beträgt.

Nicht agglomerierte Nanofüller sind an sich bekannt und z.B. in WO 0130305 A1 oder am Beispiel von SiO₂ in DE 196 17 931 A1 beschrieben. Sie können vorzugsweise ausgewählt aus der Gruppe SiO2, ZrO2, TiO₂, Al₂O₃ sowie aus Mischungen aus mindestens zwei dieser Stoffe. Sie können - wie in DE 196 17 931 A1 beschrieben - in organischen Lösungsmitteln dispergiert sein, aber auch in Wasser oder Wasser enthaltenden Lösungsmittelmischungen zugesetzt werden.

Als Dentalgläser eignen sich besonders Bariumglaspulver, vorzugsweise Bariumglas-Aluminium-Borsilikatgläser, und/oder Strontiumglaspulver. Die mittlere Partikelgrösse der grobteiligen Dentalgläser beträgt vorzugsweise 5-10 [micro]m, insbesondere um 7 [micro]m und die der feinteiligen 0,5 bis 2 [micro]m, insbesondere 1 [micro]m. Optional vorhandene weitere Dentalgläser haben z.B. mittlere Korngrössen von 2-5 oder 10-50 [micro]m.

Die Füllstoffkomponente kann demnach Dentalgläser mit insgesamt drei oder mehr Kornfraktionen aufweisen. Sie kann auch weitere, herkömmliche, auf dem Dentalgebiet übliche Füllstoffe enthalten, wie etwa Quarz-, Glaskeramik- oder Mischungen davon. Darüber hinaus können die Komposite Füllstoffe zur Erzielung einer erhöhten Röntgenopazität enthalten. Die mittlere Partikelgrösse des röntgenopaken Füllstoffs liegt vorzugsweise im Bereich von 100 bis 300 nm, insbesondere 180 bis 300 nm. Als röntgenopake Füllstoffe eignen sich z.B. die in der DE 35 02 594 A1 beschriebenen Fluoride der Seltenen Erdmetalle, d. h. die Trifluoride der Elemente 57 bis 71. Ein besonders bevorzugt verwendeter Füllstoff ist Ytterbiumfluorid, insbesondere Ytterbiumtrifluorid mit einer mittleren Partikelgrösse von etwa 300 nm. Die Menge des röntgenopaken Füllstoffs beträgt vorzugsweise 10 bis 50 Gew.-%, besonders bevorzugt 20 bis 30 Gew.-%, bezogen auf den Gesamtfüllstoffgehalt (b) im Dentalmaterial. Erfindungsgemäss wird neben den agglomerierten Oxidpartikeln mit Siliciumdioxidmatrix und Zirkoniumdioxid als Dotierkomponente vorzugsweise üblicher Füllstoff nur mit einem sehr geringen Gehalt von kleiner 5 Gew.-%, vorzugsweise kleiner 2,5 Gew.-%, bevorzugt kleiner 1,5 Gew.-% zugesetzt, insbesondere hydrophobierte silanisierte Kieselsäure.

Das Dentalmaterial umfasst vorzugsweise als härtbare Monomer- und/oder Polymerkomponente Monomere oder Polymere die nachfolgend genannten:
Als Monomere kommen die auf dem Dentalgebiet üblichen Monomere in Betracht: Beispiele sind radikalisch polymerisierbare monofunktionelle Monomere wie Mono(meth)acrylate, Methyl-, Ethyl-, Butyl-, Benzyl-, Furfuryl- oder Phenyl(meth)-acrylat, polyfunktionelle Monomere wie polyfunktionelle Acrylate bzw. Methacrylate, z.B. Bisphenol-A-di(meth)acrylat, Bis-GMA (ein Additionsprodukt aus Methacrylsäure und Bisphenol-A-diglycidylether), UDMA (Urethandimethacrylat), z.B. ein Additionsprodukt aus 2-Hydroxyethylmethacrylat und 2,2,4-Hexamethylendiisocyanat), Di-, Tri- oder Tetraethylenglykoldi(meth)acrylat, Decandioldi(meth) acrylat, Dodecandioldi-(meth)acrylat, Hexyldecandioldi(meth)acrylat, Trimethylol propantri(meth)acrylat, Pentaerythrittetra(meth)acrylat sowie Butandioldi(meth)acrylat. Besonders bevorzugt sind Bis-GMA, TEDMA (Triethylenglykoldimethacrylat), UDMA (Urethandimethacrylat), TCD-di-HEMA (Bis (methacryloyloxymethyl) tricyclo-[5.2.1.0^{2,6}]decan) und TCD-di-HEA (Bis-(acryloyloxymethyl)tricyclo[5.2.1.0^{2,6}] decan).

Als bevorzugtes Vernetzermonomer kann mindestens ein Monomer ausgewählt aus den folgenden oder Gemische dieser eingesetzt werden: 2,2-Bis-4-(3-methacryloxy-2-hydroxypropyl)-phenylpropan) (Bis-GMA), d.h. das Umsetzungsprodukt von Glycidylmethacrylat und Bisphenol-A (OH-gruppenhaltig), und 7,7,9-Trimethyl-4,13-dioxo-3,14-dioxa-5,12-diazahexadecan-1,16-diyldimethacrylat (UDMA), d.h. das Urethandimethacrylat aus 2 Mol 2-Hydroxyethylmethacrylat (HEMA) und 1 Mol 2,2,4-Trimethylhexamethylendiisocyanat (urethangruppenhaltig). Darüber hinaus sind Umsetzungsprodukte von Glycidylmethacrylat mit anderen Bisphenolen, wie z.B. Bisphenol-B(2,2'-Bis-(4-hydroxyphenyl)-butan), Bisphenol-F(2,2'-Methylendiphenol) oder 4,4'-Dihydroxydiphenyl, sowie Umsetzungsprodukte von 2 Mol HEMA oder 2-Hydroxypropyl(meth)acrylat mit, insbesondere 1 Mol, bekannter Diisocyanate, wie z.B. Hexamethylendiisocyanat, m-Xylylendiisocyanat oder Toluylendiisocyanat, als Vernetzermonomere geeignet.

Als multifunktionelle Vernetzer kommen ausser TEDMA und UDMA in Frage: Diethylenglycol-di(meth)acrylat, Decandioldi(meth)acrylat, Trimethylolpropantri-(meth)acrylat, Pentaerythrittetra(meth)acrylat sowie Butandioldi(meth)acrylat, 1,10-Decandioldi(meth)acrylat, 1,12-Dode-candioldi(meth)acrylat.

Das Dentalmaterial kann vorzugsweise als härtbare Monomer- und/oder Polymerkomponente Monomere und/oder Polymere auch die nachfolgend genannten umfassen: Ein oder mehrere ethylenisch ungesättigte Verbindungen mit oder ohne Säurefunktionalität. Beispielsweise Acrylsäureester, Methacrylsäureester, Hydroxyfunktionellen Acrylsäureester, hydroxyfunktionellen Methacrylsäureester und Kombinationen davon. Sowie Mono-, Di-oder Poly-(Meth) Acrylate, d.h. Acrylate und Methacrylate, wie Methyl(meth)acrylat, Ethylacrylat, Isopropylmethacrylat, n-Hexylacrylat, Stearylacrylat, Allylacrylat , Glycerintriacrylat, Ethylenglycoldiacrylat, Diethylenglycoldiacrylat, Triethylenglycoldimethacrylat, 1,3-Propandiol(meth)acrylat, Trimethylolpropantriacrylat, 1,2,4-Butantriol-trimethacrylat, 1,4-Cyclohexandioldiacrylat, Pentaerythrit-tetra(meth)acrylat, Sorbithexacrylate, Tetrahydrofurfuryl (meth) acrylat, Bis [1-(2-acryloxy)]-p-ethoxyphenyldimethylmethane, Bis [1-(3-acryloxy-2-hydroxy)]-p-propoxyphenyldimethylmethane, ethoxyliertes Bisphenol A-di (meth) acrylat und Trishydroxyethylisocyanurat-isocyanurat trimethacrylat, (Meth)acrylamiden (dh, Acrylamiden und Methacrylamiden), wie (Meth) acrylamid, Methylen-bis-(meth) acrylamid und Diaceton (meth) acrylamid; Urethan (meth) acrylate; die Bis-(Meth)acrylate von Polyethylenglykolen (vorzugsweise mit einem Molekulargewicht von 200-500), copolymerisierbare Mischungen von acrylierten Monomeren, und Vinylverbindungen wie Styrol, Diallylphthalat, Divinylsuccinat, Divinyladipat und Divinylphthalat. Andere geeignete radikalisch polymerisierbare Verbindungen umfassen Siloxan-funktionelle (Meth) acrylate und Fluorpolymer-funktionelle (Meth) acrylate oder Mischungen von zwei oder mehreren radikalisch polymerisierbaren Verbindungen können bei Bedarf verwendet werden.

Die polymerisierbare Komponente kann auch Hydroxylgruppen aufweisen und ethylenisch ungesättigte Gruppen in einem einzigen Molekül. Beispiele für solche Materialien umfassen Hydroxyalkyl (meth) acrylate, wie 2-Hydroxyethyl (meth) acrylat und 2-Hydroxypropyl(meth)acrylat; Glycerinmono-oder Di-(meth)acrylat; Trimethylolpropan Mono-oder Di-(Meth)acrylat ; Pentaerythrit Mono-, Di-und Tri-(meth)acrylat; Sorbitol Mono-, Di-, Tri-, Terra-oder penta-(meth) acrylat und 2,2-Bis [4 - (2-Hydroxy - 3-methacryloxypropoxy)phenyl]propan (BisGMA) oder Mischungen von ethylenisch ungesättigter Verbindungen. Die härtbare oder polymerisierbare Komponente kann PEGDMA (Polyethylenglykoldimethacrylat mit einem Molekulargewicht von etwa 400), GDMA (Glycerindimethacrylat), TEGDMA (Triethylenglycoldimethacrylat), umfassen und / oder NPGDMA (Neopentylglykol-dimethacrylat) sowie Gemische enthaltend diese.

Zur Initiierung der Polymerisation enthalten die Komposite einen Polymerisationsinitiator, beispielsweise einen Initiator für die radikalische Polymerisation. Je nach Art des verwendeten Initiators können die Mischungen kalt, durch Strahlen vernetzt, d.h. UV-vernetzt oder durch Zufuhr von Wärme polymerisierbar sein.

Als Initiatoren für die Temperatur induzierte Polymerisation können bekannte Peroxide eingesetzt werden, wie Dibenzoylperoxid, Dilauroylperoxid, tert.-Butylperoctoat oder tert.-Butylperbenzoat, aber auch alpha, alpha'-Azo-bis(isobutyro-ethylester), Benzpinakol und 2,2'-Dimethylbenzpinakol.

Als Photoinitatoren kommen beispielsweise Benzoinalkylether oder -ester, Benzilmonoketale, Acylphosphinoxide oder aliphatische und aromatische 1,2-Diketoverbindungen, wie beispielsweise 2,2-Diethoxyacetophenon 9,10-Phenanthrenchinon, Diacetyl, Furil, Anisil, 4,4'-Dichlorbenzil und 4,4'-Dialkoxybenzil oder Campherchinon, in Frage. Die Photoinitiatoren werden vorzugsweise zusammen mit einem Reduktionsmittel verwendet. Beispiele für Reduktionsmittel sind Amine wie aliphatische oder aromatische tertiäre Amine, beispielsweise N,N-Dimethyl-p-toluidin oder Triethanolamin, Cyanethylmethylanilin, Triethylamin, N,N-Dimethylanilin, N-Methyldiphenylamin, N,N-Dimethyl-sym.-xylidin, N,N-3,5-Tetramethylanilin und 4-Dimethylaminobenzoesäureethylester oder organische Phosphite. Gängige Photoinitiatorsysteme sind z.B. Campherchinon plus Ethyl-4-(N,N-dimethylamino)benzoat, 2-(Ethylhexyl)-4-(N,N-dimethylamino)benzoat oder N,N-Dimethylaminoethylmethacrylat.

Als Initiator für die durch UV-Licht initiierte Polymerisation eignet sich besonders 2,4,6-Trimethylbenzoyldiphenylphosphinoxid. UV-Photoinitiatoren können allein, in Kombination mit einem Initiator für sichtbares Licht, einem Initiator für die Kalthärtung und/oder einem Initiator für die Temperatur induzierte Härtung eingesetzt werden.

Als Initiatoren für die Kaltpolymerisation werden Radikale bildende Systeme, z. B. Benzoyl- bzw. Lauroylperoxid zusammen mit Aminen wie N,N-Dimethyl-sym.-xylidin oder N,N-Dimethyl-p-toluidin verwendet. Es können auch dual härtende Systeme verwendet werden, z. B. Photoinitiatoren mit Aminen und Peroxiden. Die Initiatoren werden vorzugsweise in Mengen von 0,01 bis 1 Gew.-% bezogen auf die Gesamtmasse der Mischung verwendet.
Bei der Kaltpolymerisation kann es zweckmässig sein, wenn das Kompositmaterial aufgeteilt in zwei Komponenten vorliegt, die zur Aushärtung durch Vermischen vorgesehen sind. Es ist auch möglich, das Material so bereitzustellen, dass es sowohl durch VIS- und/oder UV-Licht als auch durch Vermischen zweier Komponenten zu härten ist.

Gleichfalls Gegenstand der Erfindung ist ein Verfahren zur Herstellung eines erfindungsgemässen Dentalmaterials sowie ein Dentalmaterial erhältlich nach diesem Verfahren, indem (a) mindestens eine härtbare Monomer- und/oder Polymerkomponente, und (b) eine Füllstoffkomponente umfassend (b.1) mindestens agglomerierte Oxidpartikel mit einer Matrix und einer Dotierkomponente, wobei die Matrix Siliziumdioxid umfasst und die Dotierkomponente Zirkoniumdioxid, bis zu 80 Gew.-%, sowie optional (b.2) Gläser, (b.3) Rheologiemodifizierer und/oder Gemische von mindestens zwei der vorgenannten Füllstoffkomponenten, und optional (c) ein Initiator und optional (d) mindestens ein Pigment gemischt werden.

Nachfolgend kann vorteilhaft das Dentalmaterial zunächst in einem Schritt (1) geformt werden und optional in einem weiteren Schritt (2) polymerisiert und optional in einem weiteren Schritt (3) lackiert und/oder mechanisch bearbeitet werden.

Gegenstand der Erfindung ist auch ein Verfahren in dem zusätzlich als Füllstoffkomponente (b) Gläser (b.2) und/oder Gemische von mindestens zwei der vorgenannten Füllstoffkomponenten, und optional (c) ein Initiator gemischt werden. Ferner kann das Dentalmaterial (1) vorzugsweise geformt werden und (2) optional polymerisiert werden.

Erfindungsgemässe gehärtete, insbesondere durch Polymerisation erhältliche Dentalmaterialien weisen eine Transparenz von grösser gleich 55, vorzugsweise, jeweils grösser gleich 58 %, 58,5 %, 59,5 %, bevorzugt grösser gleich 60 %, besonders bevorzugt größer gleich 62 %, auf (Belichtung: 8 min Palatray CU, beidseitig, HiLite Power 180 sec. beidseitig), eine Differenz der Glanzzahl von kleiner gleich 80 nach Zahnbürstenabrasion (polierte Oberfläche bis 1000/2500/4000er Schleifpapier, Diamantsuspension gelb/rot/weiß, Zahnbürstenabrasionssimulation Willytec/SD-Mechatronik, Zahnbürste Hager & Werken, Odol-med-3, 10.000 Zyklen, Sägezahnprofil, entspricht ca. 3-6 Monaten Putzdauer) auf, insbesondere kleiner gleich 60, bevorzugt kleiner gleich 55, besonders bevorzugt kleiner gleich 50, erfindungsgemäss kleiner gleich 45, bis kleiner gleich 40. Ferner weisen die erfindungsgemässen gehärteten Dentalmaterialien jeweils unabhängig einen, mehrere oder alle der genannten Parameter auf. Erfindungsgemäss gehärtete, insbesondere durch Polymerisation erhältliche Dentalmaterialien weisen vorteilhaft eine Rauigkeit von kleiner gleich 35 µm Tiefe auf, insbesondere kleiner gleich 30 µm, besonders bevorzugt kleiner gleich 25 µm Tiefe nach Mohnabrasion auf, wie nachfolgend entsprechend den Ausführungsbeispielen ermittelt. Zusätzlich oder alternativ weisen gehärtete Dentalmaterialien eine Rauigkeit von kleiner gleich 0,4000 mm³ Volumen, insbesondere kleiner gleich 0,3500 mm³ Volumen, bevorzugt kleiner gleich 0,3000 mm³ Volumen, besonders bevorzugt kleiner gleich 0,2500 mm³ Volumen auf, ermittelt nach dem Mohnabrasionstest, Volumen in der Oberflächentextur. Erfindungsgemäss gehärtete, insbesondere durch Polymerisation erhältliche Dentalmaterialien weisen vorteilhaft eine Reflexion von grösser gleich 1,5 %, insbesondere grösser gleich 2,0 %, bevorzugt grösser gleich 2,5 %, besonders bevorzugt grösser gleich 3 %, besonders vorzugsweise grösser gleich 4,0 %, weiter bevorzugt grösser gleich 4,5 % auf, insbesondere ermittelt nach einer Zahnbürstenabrasion. Erfindungsgemäss bevorzugte, gehärtete Dentalmaterialien weisen mindestens zwei der vorgenannten Parameter bis alle Parameter auf.
Die erfindungsgemässe Füllstoffkomponente wird vorteilhaft in-situ hergestellt, indem die Füllstoffkomponente umfassend agglomerierte Oxidpartikel mit einer Matrix und einer Dotierkomponente, wobei die Matrix Siliziumdioxid enthält und die Dotierkomponente Zirkoniumdioxid umfasst, mit einem organofunktionellen Silan, wie einem ethylenisch ungesättigten organofunktionellen Silan, oberflächenmodifiziert wird. Eine bevorzugte Füllstoffkomponente umfasst agglomerierte Oxidpartikel mit Umsetzungsprodukten von olefinischen Alkoxysilanen, insbesondere von 3-Methacryloxytrimethoxysilan und/oder 3-Methacryloxytriethoxysilan oberflächenmodifiziert.

Gegenstand der Offenbarung ist auch eine Füllstoffkomponente umfassend agglomerierte Oxidpartikel mit einer Matrix und einer Dotierkomponente, wobei die Matrix Siliziumdioxid enthält und die Dotierkomponente Zirkoniumdioxid umfasst, und wobei die agglomerierten Oxidpartikel mit einem organofunktionellen Silan oberflächenmodifiziert sind. Bevorzugte Füllstoffkomponenten umfassen oberflächenmodifizierte, agglomerierte Oxidpartikel mit Umsetzungsprodukten von olefinischen Alkoxysilanen, insbesondere von 3-Methacryloxytrimethoxysilan und/oder 3-Methacryloxytriethoxysilan.

Entsprechend einem weiteren Gegenstand der Erfindung wird ein gehärtetes Dentalmaterial offenbart, dass erhältlich ist durch Polymerisation eines vorgenannten Dentalmaterials oder, das erhältlich ist durch Vermischen, optional Formen und Polymerisieren eines vorgenannten Dentalmaterials.

Ferner ist Gegenstand der Erfindung ein Dentalmaterial, das erhältlich ist durch optionales Vermischen und Formen, sowie durch Polymerisation, wobei die agglomerierten Oxidpartikel, insbesondere agglomerierten und aggregierten Oxidpartikel, in der erhaltenen Polymermatrix des Dentalmaterials eingebunden sind und bei einem Abrasionsprozess schichtweise zusammen mit der Polymermatrix des Dentalmaterials abgetragen werden und nicht als einzelne, vollständige Partikel herausbrechen.

Weiterhin ist Gegenstand der Erfindung ein Dentalmaterial erhältlich durch Polymerisation, wobei die agglomerierten und/oder aggregierten Oxidpartikel, vorzugsweise aggregierten Oxidpartikel, in einer Polymermatrix des Dentalmaterials kovalent einpolymerisiert vorliegen, vorzugsweise liegen die Oxidpartikel aggregiert und/oder kovalent in die Polymermatrix eingebunden vor, und werden bei einem Abrasionsprozess schichtweise zusammen mit der Polymermatrix des Dentalmaterials abgetragen werden und nicht als einzelne, vollständige Partikel herausbrechen. Dentalmaterial erhältlich durch Polymerisation, wobei die agglomerierten Oxidpartikel aggregiert in einer Polymermatrix des Dentalmaterials vorliegen und vorzugsweise kovalent in die Polymermatrix eingebunden sind. Dentalmaterial erhältlich durch Polymerisation, wobei die agglomerierten Oxidpartikel optional aggregiert in einer Polymermatrix des Dentalmaterials vorliegen und kovalent in die Polymermatrix eingebunden sind.

Bevorzugt ist das gehärtete bzw. polymerisierte Dentalmaterial eines der nachstehend genannten dentalen Erzeugnisse oder wird zur Herstellung dieser verwendet, umfassend: künstlicher Zahn, Verblendung, Inlay, Trägermaterial für eine lokale Freisetzung von pharmazeutisch wirksamen Substanzen, Trägermaterial für eine lokale Antibiotika-Therapie oder ein Fräsblock zur Herstellung von Zahnersatz, Zahn, Vollprothese, Brücke, insbesondere eine Brücke mit 2 bis 9 Gliedern, oder ein Fräsblock zur Herstellung von Zahnersatz nach der CAD/CAM Technik oder zumindest ein Teil der vorgenannten Dentalmaterialien ist.

Das Dentalmaterial kann zu dentalen Erzeugnissen durch Härten verarbeitet werden, beispielsweise zu Zahnfüllungen, zahnärztlichen Rohlingen, Zahnkronen und Brücken, Zahnprothesen, kieferorthopädischen Vorrichtungen und dergleichen. Dentalmaterialien schliessen zum Beispiel auch ein: Klebstoffe (z.B. Dental- und / oder kieferorthopädische Klebstoffe), Zemente (z.B. Glasionomerzemente, Harzmodifizierte Glas-Ionomer-Zemente, und/oder kieferorthopädische Zemente), Primer (z.B. kieferorthopädische Primer), Reparaturmassen, Stärkungsmittel (z. B. ein restauratives Füllmaterial), Auskleidungen, Dichtmassen (z.B. kieferorthopädische Dichtungsmittel) und Beschichtungen.

Auch kann das Dentalmaterial umfassend härtbare Monomer-und/oder Polymerkomponente in Form einer Paste oder einer formbaren Masse vorliegen, die gehärtet wird, um ein dentales Erzeugnis zu bilden. Dentale Erzeugnisse umfassen auch ein wiederhergestelltes Gebiss oder ein Teil davon. Beispiele hierfür sind Füllungsmaterialien, Zahnersatz, Inlays, Onlays, Veneers, Voll-und Teilkronen, Brücken, Implantate, Implantat-Abutments, Käppchen, anterior Füllungen, Kavitätseinlagen, Base liner, Unterfüllungsmaterial, Dichtstoffe (Beschichtung für Zähne), Zahnersatz, Brückengerüste und andere Brückenstrukturen sowie Teile davon, oder kieferorthopädische Apparaturen und Geräte und Prothesen (z. B. teilweiser oder vollständiger Zahnersatz).

Die Erfindung wird durch nachstehende Beispiele näher erläutert, ohne jedoch die Erfindung zu beschränken.
Figur 1: Stellt dar in 1 µm Auflösung die agglomerierten Oxidpartikel mit Siliziumdioxidmatrix und Dotierkomponente Zirkoniumdioxid
Figur 2: Stellt dar in 10 µm Auflösung die agglomerierten und vorzugsweise aggregierten Oxidpartikel kovalent eingebunden in dem vernetzten Dentalmaterial als abradierte Oberfläche
Figur 3: wie Figur 2 mit höherer Auflösung
Figur 4: Stellt dar in 200 nm Auflösung, die agglomerierten Oxidpartikel umfassend die Primärpartikel enthaltend die Siliziumdioxidmatrix und Zirkoniumdioxiddomänen.

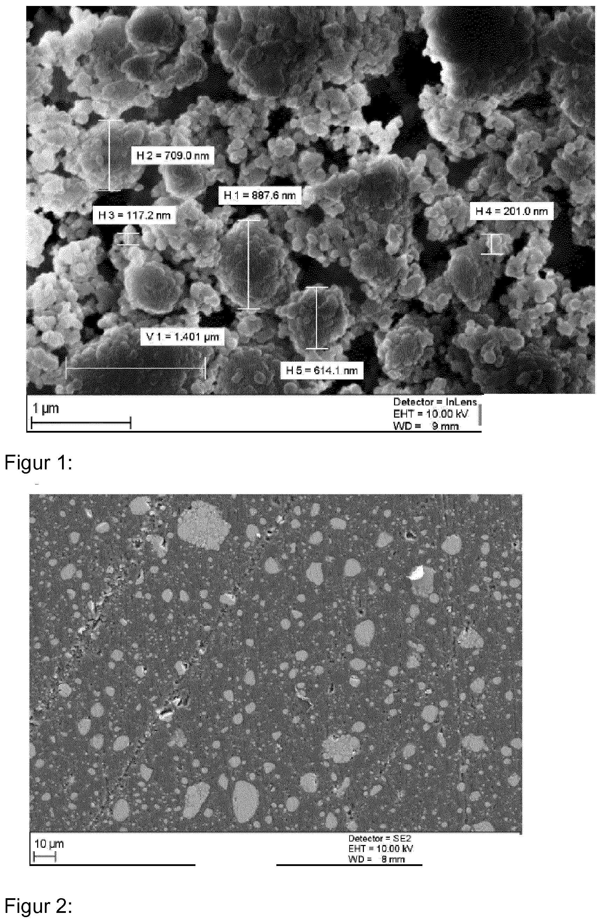

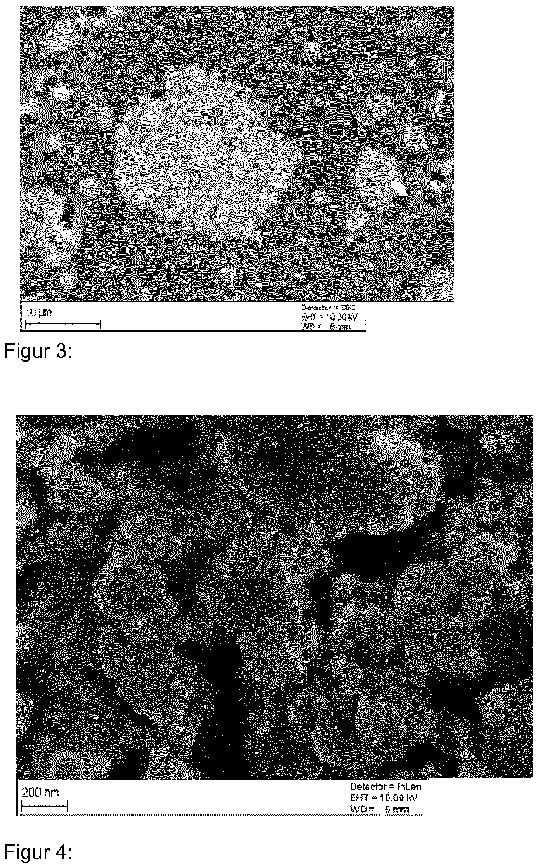

Die Figuren 2 und 3 zeigen REM-Aufnahmen des ZrO₂-dotierten Kieselsäure Füllstoffes, eingearbeitet in eine Methacrylat-Matrix, Oberfläche angeschliffen und mit Schleifpapier abnehmender Körnung poliert (bis 4000er); In Figur 3 ist deutlich die innere Struktur des Füllstoffes und das schichtweise Abtragen des Partikels durch Abrasion/Politur zu erkennen.

### Messung der Abrasionseigenschaften

Die Abbrasionseigenschaften der dentalen Materialien wurden mit Prüfmaschinen des Herstellers Willytec/SD-Mechatronik ermittelt. Generell kann ein Verschleiss von natürlichen Zähnen oder dentalen Werkstoffen durch Abrasion in-vivo durch unterschiedliche Mechanismen erfolgen, wie durch den Antagonisten - Zahn, Verschleiss durch abrasive Partikel im Speisebrei und/oder Verschleiss durch Reinigung mit Zahnbürste und/oder Zahnpasta. Diese Verschleissmechanismen können unter Laborbedingungen durch verschiedene Simulationsmethoden nachgestellt werden.

3 - Medien Abrasion (ACTA - Methode) zur Simulation von durch Speisebrei hervorgerufene Abrasion: In ein rotierendes Rad mit 12 Kammern wird das zu untersuchende Material eingefüllt und mit 180 min⁻¹ gegenläufig zu einem Antagonistenrad aus Stahl (240 min⁻¹) betrieben. Beide Räder sind in einer Suspension aus Wasser und Mohn (Mohnabrasion, MA) gelagert (110 g Mohn : 200 g Wasser). Das Antagonistenrad wird mit einer Kraft von 20 N gegen das Prüfkörperrad gedrückt. Durch den Spalt aus Prüfkörper- und Antagonistenrad können sich Mohnsamen bewegen, welche auf der Kompositoberfläche eine Oberflächenbeschädigung verursachen. Prüfmaschine 3-Medien Abrasion / Hersteller: Willytec/SD-Mechatronik

Abrasionstest: Nach 2 x 150.000 Umdrehungen des Prüfkörperrades (dies entspricht einer Tragedauer im Patientenmund von ca. 3 Jahren) wird die durch die Mohnsamen hervorgerufene Beschädigung des Komposits in jeder Kammer gemessen und nach Tiefe und Volumen mit einem Laserscanner ermittelt und ausgewertet. Die Erfassung des Tiefenprofils erfolgt berührungslos; Gemessen wird das Volumen in mm³ und die mittlere Tiefe in µm (Mikrometer).

Die Zahnbürstenabrasion dient der Simulation der Abrasion während der Reinigung durch Zahnbürste und Zahnpasta: Die Zahnbüsten (insgesamt 8 pro Prüfung) werden mit einer Kraft von 2 N auf die Prüfkörperoberfläche gedrückt und in einem Sägezahnmuster über die Prüfkörperoberfläche bewegt. Als Abrasionsmedium dient ein Zahnpasta-Wasser Gemisch (Odol-med 3, 2:1). Nach 10.000 Zyklen (dies simuliert eine Tragezeit von ca. 3 bis 6 Monaten), wird die hervorgerufene Oberflächenveränderung des Komposits gemessen und nach Rauigkeit (heute Rauheit, früher auch Rauhigkeit, Rauheit: Begriff aus der Oberflächenphysik, der die Unebenheit der Oberflächenhöhe bezeichnet) und Reflexion ausgewertet. Prüfmaschine Zahnbürstenabrasion: Hersteller Willytec/SD-Mechatronik.

Die Kausimulation (CoCoM-Methode, CoCoM-Methode/Computer Controlled Mastication) simuliert die Abrasion während einer Kaubewegung durch einen Antagonisten. Hierbei wirkt auf die Prüfkörperoberfläche eine Keramik-Kugel aus Al₂O₃ mit einer Kraft von 50 N ein. Nach dem Berühren der Prüfkörperoberfläche durch die Kugel wird der Prüfkörper um 0,8 mm seitlich verschoben und abradiert die Prüfkörperoberfläche. Ausserdem werden die Prüfkörper vor der Abprüfung einem Temperaturlastwechsel unterworfen (ca. 5000 Zyklen zwischen 5° (1 min.) / 55°C (1 min.). Pro Material werden insgesamt 16 Prüfkörper untersucht. Nach 200.000 Zyklen (simuliert Tragezeit von ca. 5 Jahren), wird die hervorgerufene Beschädigung des Komposits gemessen und nach Tiefe und Volumen ausgewertet. Prüfmaschine Kausimulation: Hersteller Willytec/SD-Mechatronik; Gemessen wird das Volumen in mm³ und die Tiefe in µm (Mikrometer).

### Glanzzahl: Byk-Gardner Glanzmeßgerät Tri-Gloss, Meßwinkel 60°

Nachfolgen werden die erfindungsgemäßen vorteilhaften Eigenschaften anhand den erfindungsgemäßen Beispiele 1 bis 4 gegenüber den Vergleichsbeispielen VG1, VG2, VG3, VG4) aufgezeigt. Die in einer Tabelle zusammengefassten Bespiele wurden jeweils soweit sie die gleichen Komponenten oder Verbindungen enthalten mit im Wesentlichen gleichen Mengen der Komponenten oder Verbindungen durchgeführt. Die Bestandteile der Zusammensetzungen sind immer auf 100 Gew.-% (Gesamtzusammensetzung) bezogen.

**Tabelle 1:**

| **Ausführungsbeispiel: Grundmasse** | | VG1 | 1 |
|---|---|---|---|
| Monomere | Bisglycidyl acrylat | 3-7 | 3- 7 |
| | Urethandimethacrylat (UDMA)) | 10-15 | 10 - 15 |
| | alkoxyliertes Pentaerythritol tetraacrylat | 7-10 | 7-10 |
| | TEDMA (Triethylenglykoldimethacrylat) | < 3 | < 3 |
| Initiatoren | Butylhydroxytoluol | < 1 | <1 |
| | DL-Campherchinon | <1 | <1 |
| | Benzildimethylketal | < 1 | < 1 |
| | tert. Amin | < 0,5 | <0,5 |
| Rheologie modifizierer | Schichtkieselsäure | 14 - 18 | X |
| | Nanoteilige Kieselsäure (trimethylsilyl oxymod ifiziert | X | < 2 |
| | (d50: 2,6 µm) ZrO₂-SiO₂ (d50: 2,6 µm) | X | 20 - 25 |
| | Gamma-methacryloxypropyl- trimethoxy silan | < 3 | < 3 |
| Füllstoffe | Glaspulver unsil. (Barium aluminium bor silicat glas) | 50-60 | X |
| | Glaspulver sil. (Barium aluminium bor silicat glas) | X | 45-50 |

**Tabelle 2:**

| Verbesserung der Abrasionsbeständigkeit: DA3 | | VG2 | 2 |
|---|---|---|---|
| Monomere | Bisglycidyl acrylat | 3 - 7 | 3-7 |
| | UDMA Urethandimethacrylat | 10 - 15 | 10 - 15 |
| | alkoxyliertes Pentaerythritol tetraacrylat | 7 - 10 | 7-10 |
| | TEDMA Triethylenglykoldimethacrylat | < 3 | < 3 |
| Initatoren | Butylhydroxytoluol | < 1 | < 1 |
| | DL-Campherchinon | < 1 | < 1 |
| | Benzildimethylketal | < 1 | < 1 |
| | tert. Amin | < 1 | < 1 |
| Rheologie modifizierer | Schichtkieselsäure | 14 - 18 | X |
| | Nanoteilige Kieselsäure (trimethylsilyl oxymod ifiziert) | X | < 2 |
| | ZrO₂-SiO₂ (d₅₀ 2,6 µm) | X | 20 - 25 |
| | Gamma-methacryloxypropyl- trimethoxy silan | < 3 | < 3 |
| Füllstoffe | Glaspulver unsil. (Barium aluminium bor silicat glas) | 50 - 60 | X |
| | Glaspulver sil. (Barium aluminium bor silicat glas) | X | 45-50 |
| | Pigmente (total) | < 1 | < 1 |

Durch die Verwendung des erfindungsgemässen Füllstoffes ergeben sich deutliche Verbesserungen in Zahnbürstenabrasion (ZB), Mohnabrasion (MA) und Kaumaschinenabrasion (KM).

**Tabelle 3:**

| | | | |
|---|---|---|---|
| Abrasionsmessungen | ZB, Tiefe (µm, Mikrometer) | 1,29 | 1.04 |
| | ZB, Refl (%) | 2,6 | 4,6 |
| | Glanzzahl (Differenz Vorher Nachher) | 87,9 | 39,5 |
| | MA, Tiefe (µm, Mikrometer) | 42,6 | 24,1 |
| | MA, Vol (mm³) | 0,4294 | 0,2213 |
| | KM neu, Tiefe (µm) | 151,7 | 134,3 |
| | KM neu, Vol (mm³) | 0,2108 | 0,1623 |

**Tabelle 4:**

| | VG3 | 3 | 4 | VG4 |
|---|---|---|---|---|
| Bisglycidyl acrylat | 3 - 7 | 3 - 7 | 3 - 7 | 3 - 7 |
| UDMA Urethandimethacrylat | 10 - 15 | 10 - 15 | 10 - 15 | 10 - 15 |
| alkoxyliertes Pentaerythritol tetraacrylat | 6 - 10 | 6 - 10 | 6 - 10 | 6 - 10 |
| TEDMA | < 3 | < 3 | < 3 | < 3 |
| DL-Campherchinon | < 1 | < 1 | < 1 | < 1 |
| Butylhydroxytoluol | < 1 | < 1 | < 1 | < 1 |
| Benzildimethylketal | < 1 | < 1 | < 1 | < 1 |
| tert. Amin | < 1 | < 1 | < 1 | < 1 |
| ZrO₂-SiO₂ (d₅₀ 2,6 µm) | | 18 - 25 | 11 - 15 | |
| Merck JE340 4-8 µm (Mikrometer) | | | | 14 |
| Gamma-methacryloxypropyl-trimethoxy silan | < 3 | < 3 | < 3 | |
| Nanoteilige Kieselsäure (trimethylsilyl oxymod ifiziert) | | | < 2 | < 2 |
| Sch ichtkieselsäure | 14 - 18 | | | |
| Glaspulver sil. (Barium aluminium bor silicat glas) | 50 - 65 | 50-65 | 50-65 | 50-65 |
| Gew.-% | 100 | 100 | 100 | 100 |

**Tabelle 5:**

| | | VG3 | 3 | 4 | VG4 |
|---|---|---|---|---|---|
| 3-Punkt Biege [MPa] (HiLite Power 180/90 sec. beidseitig) | | 141,2 | 142,4 | 139,1 | 147 |
| | | 10224 | 10919 | 9024 | 10720,0 |
| Farbwerte (8 min Palatray CU - beidseitige Belichtung.) (HiLite Power 180 sec. beidseitig) | L (Helligkeit) | 91,12 | 91,43 | 91,58 | 87,21 |
| | a (rot/grün) | -2,75 | -1,01 | -1,43 | -1,88 |
| | b (gelb/blau) | 13,73 | 6,22 | 6,17 | 10,13 |
| | C (Chroma) | 14 | 6,3 | 6,34 | 10,3 |
| | h (Farbton) | 101,32 | 99,19 | 103,03 | 100,49 |
| | T (Transparenz) [%] | 57,2 | 64,15 | 59,44 | 41,16 |
| Zahnbürste Reflexion [%] | | 4,70 | 7,90 | 7,60 | 7,30 |
| Zahnbürste Rauheit [µm] | | 0,5200 | 0,38 | 0,49 | 0,54 |
| MA Tiefe (µm) | | 39,80 | | 20,9 | |
| MAVol. (mm³) | | 0,3886 | | 0,1753 | |

### Glanzzahl: Byk-Gardner Glanzmeßgerät Tri-Gloss, Meßwinkel 60°

Durch die Verwendung des erfindungsgemässen Füllstoffes ergeben sich deutliche Verbesserungen in der Transparenz der ungefärbten Grundmasse. Siliziumdioxid du Zirkondioxid enthaltende (ZrO₂-SiO₂)-Produkte anderer Hersteller zeigen diese positiven Effekte nicht.

## Patentansprüche

1. Dentalmaterial, umfassend
(a) mindestens eine härtbare Monomer- und/oder Polymerkomponente und
(b) mindestens eine Füllstoffkomponente, umfassend
(b.1) 5 bis 30 Gew.-% mindestens agglomerierte Oxidpartikel mit einer Matrix und einer Dotierkomponente, wobei die Matrix Siliziumdioxid umfasst und die Dotierkomponente Zirkoniumdioxid,
(b.2) 30 bis 65 Gew-% dentale Gläser,
(b.3) 0 bis 5 Gew.-% Rheologiemodifizierer,
oder Gemische der vorgenannten Füllstoffkomponenten, wobei
(b) die mindestens eine Füllstoffkomponente agglomerierte Oxidpartikel mit einer Matrix und einer Dotierkomponente umfasst, wobei die Matrix Siliziumdioxid enthält und die Dotierkomponente Zirkoniumdioxid umfasst, wobei agglomerierten Oxidpartikel in Bezug auf ihre Gesamtzusammensetzung 1 bis 25 Gew.-% Zirkoniumdioxid enthalten,
wobei die agglomerierten Oxidpartikel Agglomerate von Siliziumdioxid Primärteilchen umfassen, die mit Zirkoniumdioxid dotiert sind, wobei die Agglomerate eine Partikelgrösse von grösser gleich 0,1 bis kleiner gleich 12 µm aufweisen, und, wobei die Primärteilchen im Durchschnitt mindestens einen Partikeldurchmesser von circa 3 bis 70 nm aufweisen und die Primärteilchen mikrokristalline Domänen enthaltend Zirkoniumdioxid von 4 bis 7 nm umfassen.

2. Dentalmaterial nach Anspruch 1, **dadurch gekennzeichnet, dass** die agglomerierten Oxidpartikel in Bezug auf ihre Gesamtzusammensetzung 75 bis 80 Gew.-% Siliziumdioxid und 20 bis 25 Gew.-% Zirkoniumdioxid aufweisen.

3. Dentalmaterial nach einem der Ansprüche 1 oder 2, **dadurch gekennzeichnet, dass** der Brechungsindex der agglomerierten Oxidpartikel zwischen 1,49 bis 1,55 beträgt.

4. Dentalmaterial nach einem der Ansprüche 1 bis 3, **dadurch gekennzeichnet, dass** die Füllstoffkomponente agglomerierte Oxidpartikel umfassend Agglomerate von Primärteilchen mit Siliziumdioxid und Zirkoniumdioxid umfasst, wobei die Agglomerate eine Partikelgrösse d₅₀ von 0,5 bis 10 µm aufweisen.

5. Dentalmaterial nach einem der Ansprüche 1 bis 4, **dadurch gekennzeichnet, dass** die agglomerierten Oxidpartikel eine Korngrössenverteilung aufweisen mit d₉₀ kleiner gleich 12 µm und eine mittleren Partikelgrösse d₅₀ etwa zu 2,4 bis 3,0 µm.

6. Dentalmaterial nach einem der Ansprüche 1 bis 5, **dadurch gekennzeichnet**, (b) die Füllstoffkomponente ein Gemisch von (b.1), (b.2) und (b.3) enthält.

7. Dentalmaterial nach einem der Ansprüche 1 bis 6, **dadurch gekennzeichnet, dass** es (c) mindestens einen Initiator enthält.

8. Dentalmaterial nach einem der Ansprüche 1 bis 7, **dadurch gekennzeichnet, dass** (a) die agglomerierten Oxidpartikel, (b) die agglomerierten und aggregierten Oxidpartikel und/oder (c) oberflächenmodifizierte agglomerierte Oxidpartikel, jeweils (a), (b) oder (c) unabhängig, nicht durch hohe Scherkräfte, die während der Herstellung von Dentalmaterialien auftreten, in die Primärpartikel aufgespalten werden.

9. Dentalmaterial nach einem der Ansprüche 1 bis 8, **dadurch gekennzeichnet, dass** die agglomerierten Oxidpartikel einen Kristallinitätsindex von 0,6 bis 0,7 aufweisen.

10. Verfahren zur Herstellung eines Dentalmaterials nach einem der Ansprüche 1 bis 9, **dadurch gekennzeichnet, dass**
(a) mindestens eine härtbare Monomer- und/oder Polymerkomponente, und
(b) eine Füllstoffkomponente nach Anspruch 1 gemischt werden.

11. Gehärtetes Dentalmaterial, erhältlich durch Polymerisation eines Dentalmaterials nach einem der Ansprüche 1 bis 9 oder erhältlich durch Vermischen, optional Formen und Polymerisieren eines Dentalmaterials nach einem der Ansprüche 1 bis 9.

12. Gehärtetes Dentalmaterial nach Anspruch 11 mit einer Transparenz von grösser 58 (Belichtung : 8 min Palatray CU beidseitig., HiLite Power 180 sec. beidseitig.), einer Differenz der Glanzzahl von kleiner gleich 45 nach Zahnbürstenabrasion (polierte Oberfläche bis 1000/2500/4000er Schleifpapier, Diamantsuspension gelb/rot/weiß, Zahnbürstenabrasionssimulation Willytec/SD-Mechatronik, Zahnbürste Hager & Werken, Odol-med-3, 10.000 Zyklen, Sägezahnprofil, entspricht ca. 3-6 Monaten Putzdauer), einer Rauigkeit von kleiner 30 µm Tiefe nach Mohnabrasion, einer Rauigkeit von kleiner gleich 0,3500 mm³ Volumen und/oder mit einer Reflexion von grösser 2.5 % ermittelt nach einer Zahnbürstenabrasion oder ein Dentalmaterial, wobei das Dentalmaterial mindestens zwei der vorgenannten Parameter aufweist.

13. Gehärtetes Dentalmaterial nach Anspruch 11 oder 12, **dadurch gekennzeichnet, dass** die agglomerierten und optional aggregierten Oxidpartikel in einer Polymermatrix des Dentalmaterials eingebunden sind und bei einem Abrasionsprozess schichtweise zusammen mit der Polymermatrix des Dentalmaterials abgetragen werden und nicht als einzelne, vollständige Partikel herausbrechen.

14. Extra-Orale Verwendung eines Dentalmaterials nach einem der Ansprüche 1 bis 9 oder hergestellt nach Anspruch 10 zur Herstellung eines Füllungskomposits, Verblendkomposits, Verblendung, künstlichen Zahns, Inlays, Zemente, Zahnersatz, zur Herstellung von Trägermaterialien für eine lokale Antibiotika-Therapie oder als Trägermaterial für eine lokale Freisetzung von pharmazeutisch wirksamen Substanzen, Fräsblocks zur Herstellung von Zahnersatz nach der CAD/CAM Technik.

## Claims

1. Dental material comprising
a) at least one curable monomer component and/or polymer component, and
b) at least one filler component comprising
(b.1) 5 to 30 % by weight at least agglomerated oxide particles having a matrix and a doping component, wherein the matrix comprises silicon dioxide and the doping component comprises zirconium dioxide,
(b.2) 30 to 65 % by weight dental glasses,
(b.3) 0 to 5 % by weight rheology modifier,
or mixtures of the aforementioned filler components, wherein
b) the at least one filler component comprises agglomerated oxide particles having a matrix and a doping component, wherein the matrix contains silicon dioxide and the doping component comprises zirconium dioxide, wherein the agglomerated oxide particles contain 1 to 25 % by weight zirconium dioxide, based on the total composition,
wherein the agglomerated oxide particles comprise agglomerates of silicon dioxide primary particles doped with zirconium dioxide, wherein the agglomerates have a particle size of greater than or equal to 0.1 µm to less than or equal to 12 µm, and wherein the primary particles have at least an average particle diameter of approximately 3 to 70 mm, and the primary particles comprise micro-crystalline domains containing zirconium dioxide of 4 to 7 nm.

2. Dental material according to claim 1, **characterised in that** the agglomerated oxide particles have 75 to 80 % by weight silicon dioxide and 20 to 25 % by weight zirconium dioxide, based on their total composition.

3. Dental material according to any one of the claims 1 or 2, **characterised in that** the refractive index of the agglomerated oxide particles is between 1.49 and 1.55.

4. Dental material according to any one of the claims 1 to 3, **characterised in that** the filler component comprises agglomerated oxide particles comprising agglomerates of primary particles with silicon dioxide and zirconium dioxide, wherein the agglomerates have a particle size d₅₀ of 0.5 to 10 µm.

5. Dental material according to any one of the claims 1 to 4, **characterised in that** the agglomerated oxide particles have a grain size distribution with d₉₀ of less than 12 µm and a mean particle size d₅₀ of approximately 2.4 to 3.0 µm.

6. Dental material according to any one of the claims 1 to 5, **characterised in that** (b) the filler component contains a mixture of (b.1), (b.2) and (b.3).

7. Dental material according to any one of the claims 1 to 6, **characterised in that** it comprises (c) at least one initiator.

8. Dental material according to any one of the claims 1 to 7, **characterised in that** (a) the agglomerated oxide particles, (b) the agglomerated and aggregated oxide particles, and/or (c) the surface-modified agglomerated oxide particles, (a), (b) or (c) each independently, are not cleaved into the primary particles by high shear forces occurring during the production of dental materials.

9. Dental material according to any one of the claims 1 to 8, **characterised in that** the agglomerated oxide particles have an index of crystallinity of 0.6 to 0.7.

10. Method for the production of a dental material according to any one of the claims 1 to 9, **characterised in that**
(a) at least one curable monomer component and/or polymer component, and
(b) a filler component according to claim 1 are being mixed.

11. Cured dental material, obtainable by polymerisation of a dental material according to any one of the claims 1 to 9, or obtainable by mixing, optionally forming and polymerizing a dental material according to any one of the claims 1 to 9.

12. Cured dental material according to claim 11, having a transparency of greater than 58 (exposure: 8 min Palatray CU, on both sides, HiLite Power 180 sec, on both sides), a gloss level difference of less than or equal to 45 after toothbrush abrasion (polished surface up to 1000/2500/4000-grit polishing paper, diamond suspension yellow/red/white, toothbrush abrasion simulation, Willytec/SD-Mechatronik, toothbrush Hager & Werken, Odol-med-3, 10,000 cycles, sawtooth profile, corresponds to approx. 3-6 month of brushing), a roughness of less than 30 µm depth after poppy abrasion, a roughness of less than or equal to 0.3500 mm³ volume, and/or having a reflection of greater than 2.5 % as determined after a toothbrush abrasion, or a dental material, wherein the dental material has at least two of the aforementioned parameters.

13. Cured dental material according to claim 11 or 12, **characterised in that** the agglomerated and, optionally, aggregated oxide particles are incorporated into a polymer matrix of the dental material, and are removed layer-by-layer together with the polymer matrix of the dental material during an abrasion process and do not chip away as individual intact particles.

14. Extra-oral use of a dental material according to any one of the claims 1 to 9 or produced according to claim 10 for the production of a filling composite, veneering composite, veneer, artificial tooth, inlays, cements, dental prostheses, for the production of carrier materials for local antibiotic therapy, or as carrier material for local release of pharmaceutically effective substances, milling blocks for the production of dental prostheses according to the CAD/CAM technology.

## Revendications

1. Matière dentaire comprenant
a) au moins un composant durcissable de monomère et/ou de polymère, et
b) au moins un composant de charge, comprenant
(b.1) 5 à 30 % en poids au moins des particules d'oxide agglomérées ayant une matrice et un composant de dopage , la matrice comprenant le dioxyde de silicium et le composant de dopage comprenant le dioxyde de zirconium,
(b.2) 30 à 65 % en poids des verres dentaire,
(b.3) 0 à 5 % en poids des modificateurs de rhéologie,
ou des mélanges des composants de charge susmentionnés,
b) l'au moins un composant de charge comprenant des particules d'oxyde agglomérées ayant une matrice et un composant de dopage, la matrice contenant le dioxyde de silicium et le composant de dopage comprenant le dioxyde de zirconium, les particules d'oxyde agglomérées contenant 1 à 25 % en poids le dioxyde de zirconium, sur la base de leur composition totale,
les particules d'oxyde agglomérées comprenant des agglomérés des particules primaires du dioxyde de silicium dopées avec le dioxyde de zirconium, les agglomérés avant une taille des particules de supérieure ou égal à 0,1 µm à inférieure ou égal à 12 µm, et les particules primaires avant au moins un diamètre moyen des particules d'environ 3 à 70 nm, et les particules primaires comprenant des domaines microcristallines contenant le dioxyde de zirconium de 4 à 7 nm.

2. Matière dentaire selon la revendication 1, **caractérisée en ce que** les particules d'oxyde agglomérées ont 75 à 80 % en poids du dioxyde de silicium et 20 à 25 % en poids du dioxyde de zirconium, sur la base de leur composition totale.

3. Matière dentaire selon l'une des revendications 1 ou 2, **caractérisée en ce que** l'indice de réfraction des particules d'oxyde agglomérés est entre 1,49 et 1,55.

4. Matière dentaire selon l'une des revendications 1 à 3, **caractérisée en ce que** le composant de charge comprend des particules d'oxyde agglomérés comprenant des agglomérés des particules primaires avec le dioxyde de silicium et le dioxyde de zirconium, les agglomérés avant une taille des particules d₅₀ de 0,5 à 10 µm.

5. Matière dentaire selon l'une des revendications 1 à 4, **caractérisée en ce que** les particules d'oxyde agglomérées ont une distribution de granulométrie avec d₉₀ d'inférieure à 12 µm et une taille moyenne des particules d₅₀ d'environ 2,4 à 3,0 µm.

6. Matière dentaire selon l'une des revendications 1 à 5, **caractérisée en ce que** (b) le composant de charge contient un mélange de (b.1), (b.2) et (b.3).

7. Matière dentaire selon l'une des revendications 1 à 6, **caractérisée en ce qu'**elle comprend (c) au moins un initiateur.

8. Matière dentaire selon l'une des revendications 1 à 7, **caractérisée en ce que** (a) les particules d'oxyde agglomérées, (b) les particules d'oxyde agglomérés et agrégées, et/ou (c) les particules d'oxyde modifiées en surface, (a), (b) ou (c) chacun indépendamment, ne sont pas clivées en les particules primaires par des forces de cisaillement élevées survenant pendant la production des matières dentaires.

9. Matière dentaire selon l'une des revendications 1 à 8, **caractérisée en ce que** les particules d'oxyde agglomérées ont un indice de cristallinité de 0,6 à 0,7.

10. Procédé pour la production d'une matière dentaire selon l'une des revendications 1 à 9, **caractérisé en ce que**
(a) au moins un composant durcissable de monomère et/ou de polymère, et
(b) un composant de charge selon la revendication 1 sont mélangés.

11. Matière dentaire durcie, obtenable par la polymérisation d'un matière dentaire selon l'une des revendications 1 à 9, ou obtenable par mélangeant, facultativement formant et polymérisant une matière dentaire selon l'une des revendication 1 à 9.

12. Matière dentaire durcie selon la revendication 11, ayant une transparence de supérieure à 58 (exposition : 8 min Palatray CU, de part et d'autre, HiLite Power 180 sec, de part et d'autre), une différence du degré de brillance d'inférieure ou égal à 45 après l'abrasion de brosse à dents (surface polie jusqu'à 1000/2500/4000, suspension diamantée jaune/rouge/blanche, simulation d'abrasion de brosse à dents, Willytec/SD-Mechatronik, brosse à dents Hager & Werken, Odol-med-3, 10.000 cycles, profil en dents de scie, correspond à env. 3-6 mois de brossant), une rugosité d'inférieure à 30 µm profondeur après l'abrasion de pavot, une rugosité d'inferieure ou égal à 0,3500 mm³ volume, et/ou ayant une réflexion de supérieure à 2,5 % déterminée après une abrasion de brosse à dents, ou une matière dentaire, la matière dentaire avant au moins deux des paramètres susmentionnés.

13. Matière dentaire durcie selon la revendication 11 ou 12, **caractérisée en ce que** les particules d'oxyde agglomérées et facultativement agrégées sont incorporées dans une matrice de polymère de la matière dentaire, et sont enlevées couche-par-couche conjointement avec la matrice de polymère de la matière dentaire pendant un processus d'abrasion et ne s'écaillent pas en tant que des particules individuelles intactes.

14. Utilisation extra-orale d'une matière dentaire selon l'une des revendications 1 à 9 ou produite selon la revendication 10 pour la production d'un composite obturant, un composite facettant, la facette, la dent artificielle, des inlays, de ciments, des prothèse dentaires, pour la productions des matières de support pour une antibiothérapie locale, ou en tant qu'une matière de support pour la libération des substances pharmaceutiquement efficaces, des blocs de fraisage pour la production des prothèses dentaires selon la technologie CAO/FAO.
